# EUROPEAN PATENT SPECIFICATION

(11) **EP 3 056 491 B1**
(45) Date of publication and mention of the grant of the patent: **08.08.2018**
(21) Application number: 14851652.9
(22) Date of filing: 11.10.2014
(51) Int. Cl.: C07D 333/72, C07F 7/18

(54) **SYNTHETIC INTERMEDIATE OF MAXACALCITOL, PREPARATION METHOD THEREFOR AND USE THEREOF**
SYNTHETISCHES ZWISCHENPRODUKT VON MAXACALCITOL, HERSTELLUNGSVERFAHREN UND VERWENDUNG DAVON
INTERMÉDIAIRE SYNTHÉTIQUE DU MAXACALCITOL, SON PROCÉDÉ DE PRÉPARATION ET UTILISATION ASSOCIÉE

(30) Priority: 12.10.2013 CN 201310475989
(43) Date of publication of application: 17.08.2016
(73) Proprietor: Zhejiang Hisun Pharmaceutical Co. Ltd., Zhejiang 318000 (CN)
(72) Inventor: ZHENG, Guojun, Shanghai 201612 (CN); WANG, Yaping, Shanghai 201612 (CN); FENG, Shi, Shanghai 201612 (CN)
(74) Representative: HGF Limited
(86) International application number: PCT/CN2014/088336
(87) International publication number: WO 2015/051762

(56) References cited:
- WO-A2-2012/122451
- CN-A- 102 796 134
- CN-A- 103 508 999
- JP-A- 2011 157 326
- HITOSHI SHIMIZU ET AL: "Industrial Synthesis of Maxacalcitol, the Antihyperparathyroidism and Antipsoriatic Vitamin D3 analogue Exhibiting Low Calcemic Activity", ORGANIC PROCESS RESEARCH AND DEVELOPMENT, AMERICAN CHEMICAL SOCIETY, US, vol. 9, no. 3, 20 May 2004 (2004-05-20), pages 278-287, XP002675913, ISSN: 1083-6160, DOI: 10.1021/OP049822X [retrieved on 2005-04-29]
- TSUYOSHI YAMAUCHI ET AL: "Total Inversion of Cis-C5 Maxacalcitol into Its Trans-C5 Isomer via the Sulforene Intermediate", HETEROCYCLES COMMUNICATION | SPECIAL ISSUE, vol. 68, no. 12, 31 October 2006 (2006-10-31), page 2509, XP055349710, JP ISSN: 0385-5414, DOI: 10.3987/COM-06-10875
- FENG, SHI ET AL.: 'Improved and Efficient Synthesis of Maxacalcitol' CHINESE CHEMICAL LETTERS vol. 25, no. 5, 31 May 2014, page 774, XP029023917

## Description

The application claims priority of Chinese Patent Application CN201310475989.7 filed on October 12, 2013.

### Field of invention

The present invention relates to a preparation method of a drug, specifically, the present invention relates to a preparation method of Maxacalcitol, a novel synthetic intermediate thereof, a preparation method and a use therefor.

### Prior arts

Maxacalcitol (Maxacalcitol, CAS NO.: 103909-75-7), whose English chemical formula is: 22-Oxacalcitriol; (1R,3S,5Z)-4-Methylene-5-[(2E)-2-[(1S,3aS,7aS)-octahydro-1-[(1S)-1-(3-hydroxy-3-Methylbutoxy)ethyl]-7a-Methyl-4H-inden-4-ylidene]ethylidene]-1,3-cyclohexanediol, is the third generation of active vitamin D3 drug developed by Chugai Pharmaceutical Co., Ltd., and first faced to the market in Japan in 2000, its injection (Trade name: Oxarol) is used for treating the secondary hyperparathyroidism of the renal dialysis patients (SHPT); its ointment (Trade name: Oxarol) is used for treating the dry tinea skin diseases such as psoriasis. Currently, applications involving its synthesis include WO2012/122451, WO2001079166, US5436401, CN102796134 and JP20111573261.

US5436401A discloses a preparation method of Maxacalcitol, in which 1α-hydroxyl dehydroepiandrosterone is used as a starting material, and Maxacalcitol is given through modification on side chain and ring A, opening ring B by photochemical reaction and rearrangement under heating condition. However, 1α-hydroxyl dehydroepiandrosterone is prepared by microbial fermentation, which greatly restricts the source of the starting material, and the preparation method involves multiple reaction steps, some of which have relative low yields, which is not suitable for industrial production.

WO2012/122451 improves the preparation method of Maxacalcitol greatly and reduces the reaction steps by introducing a product as the starting material which is obtained by proper modifying an analog compound of vitamin D2. However, the improved method employs NaBH₄ when reducing the ketone at C-20 position, the main product of which is with opposite configuration, this greatly restricts the application of the process.

CN102796134 aims mainly at the shortage of the process in WO2012/122451, focuses on improving the reduction of the ketone at C-20 position disclosed in WO2012/122451, and obtains the product with single configuration through asymmetric reduction. JP2011157326 takes vitamin D2 as the starting material, and obtains

compound X according to the method in US4866048, the compound X is converted into compound V' (S configuration) and V"(R configuration) with a ratio of 35:65 under the action of lithium aluminium hydride, the compound V'(S configuration) is the target configuration (with a yield of 24% only), the synthesis efficiency is too low.

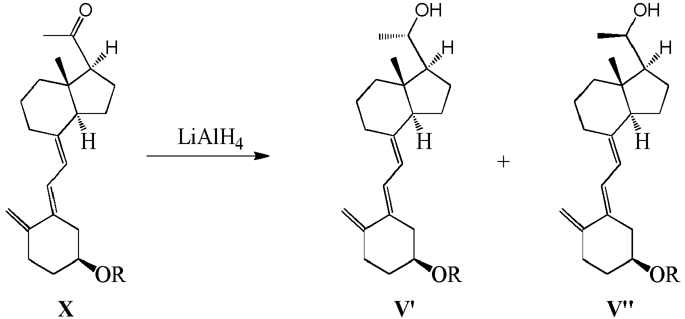

In view of the shortcomings in the prior art, it's extremely important to find a synthesis process with fewer steps, higher yield and lower cost.

### Content of the present invention

One of the aims of the present invention is to provide a novel key intermediate (compound III, IV, VI) and preparation method thereof.

Another aim of the present invention is to provide a novel preparation method of Maxacalcitol by using the key intermediate.

One aspect of the present invention is to provide a novel intermediate represented by Formula III used for the synthesis of Maxacalcitol: 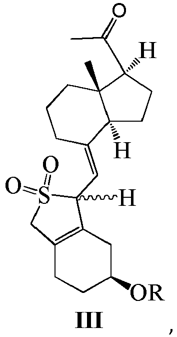 where R is H or a hydroxyl protection group, wherein the hydroxyl protection group includes a silicon ether protection group, namely a *t*-butyldimethylsilyl, a trimethylsilyl, a triethylsilyl, a *t*-butyldiphenylsilyl or a triisoprolylsilyl.
Another aspect of the present invention is to provide a preparation method of compound III, comprising in the presence of a catalyst, oxidating compound II with an
oxidizing agent to afford compound III, where R is defined as above:

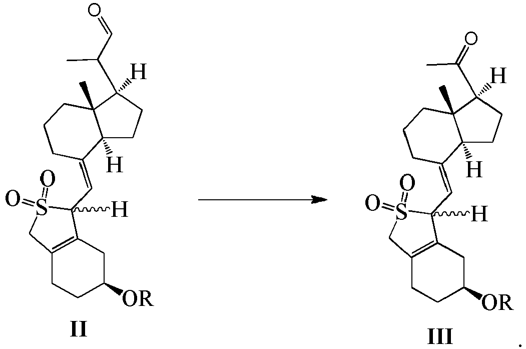

As a preferred embodiment of the present invention, the oxidizing agent of the oxidation reaction is preferably oxygen; the catalyst is preferably a copper catalyst, more preferably 2,2-bipyridine copper complex.

Another aspect of the present invention is to provide a novel intermediate represented by Formula IV used for the synthesis of Maxacalcitol: 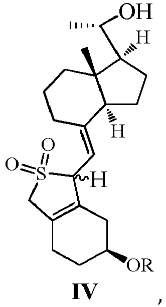 where R is H or a hydroxyl protection group, wherein the hydroxyl protection group comprises a silicon ether protection group, namely a *t*-butyldimethylsilyl, a trimethylsilyl, a triethylsilyl, a *t*-butyldiphenylsilyl or a triisoprolylsilyl.

Another aspect of the present invention is to provide a preparation method of compound IV, comprising:
in the presence of a chiral auxiliary reagent, stereoselectively reducing compound III to give compound IV with specific configuration by employing a borane, where R is defined as above:

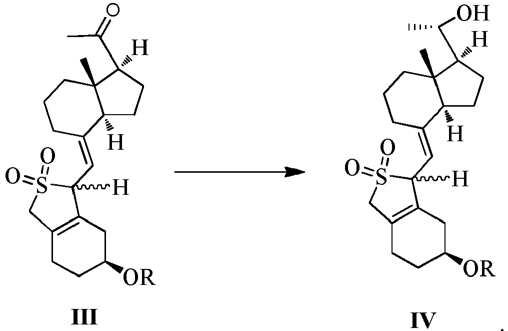

As a preferred embodiment of the present invention, the chiral auxiliary reagent used in the reaction is preferably selected from (*R*)-2-methyl-CBS-oxazaborolidine, (*R*)-2-ethyl-CBS-oxazaborolidine or (*R*)-2-isopropyl-CBS-oxazaborolidine; the borane used in the reaction is preferably selected from BH₃, borane-tetrahydrofuran complex, borane-triethylamine complex, borane-ethyl ether complex, borane-methyl sulfide complex or borane-*N,N-*diethylaniline complex.

As a preferred embodiment of the present invention, a mole ratio of the compound III, the chiral auxiliary reagent and the borane is preferably 1:0.1∼1:1∼2, more preferably 1:0.6:1.

As a preferred embodiment of the present invention, the reaction temperature is preferably -60°C to 0°C, more preferably -20°C.

Another aspect of the present invention is to provide a novel intermediate represented by Formula VI for the synthesis of Maxacalcitol: 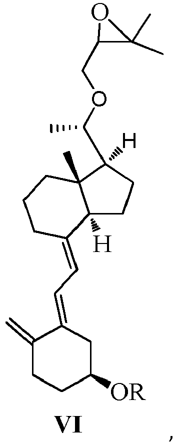 where R is H or a hydroxyl protection group, wherein the hydroxyl protection group includes a silicon ether protection group, namely a *t*-butyldimethylsilyl, a trimethylsilyl, a triethylsilyl, a *t*-butyldiphenylsilyl or a triisoprolylsilyl.

Another aspect of the present invention is to provide a preparation method of compound VI, comprising:
Step 1: converting compound IV into compound V under alkaline condition: 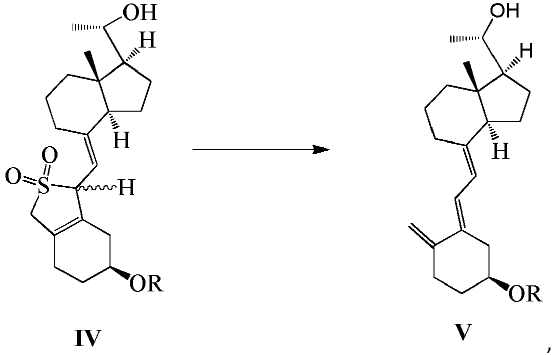 where R is a hydroxyl protection group;
Step 2: reacting compound V with 3-bromomethyl-2,2-dimethyloxirane to give compound VI: 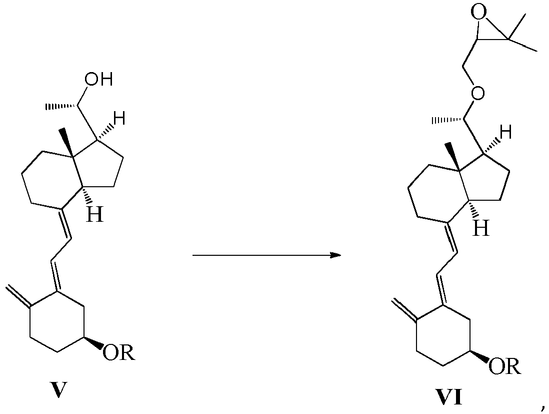 where R is a hydroxyl protection group.

The preparation method of compound VI, if it is necessary, can further comprises: de-protecting the hydroxyl protection group R of compound VI which is obtained in the step 2 to give compound VI: 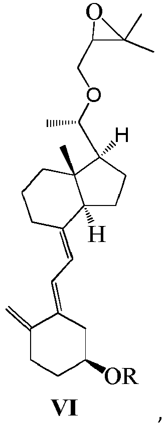 where R is H.

Wherein, the alkali in the step 1 includes sodium bicarbonate or sodium acetate.

Another aspect of the present invention is to provide a preparation method of Maxacalcitol represented by formula I:

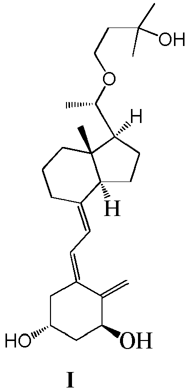

The preparation method comprises:
Step 1: converting compound IV into compound V under alkaline condition: 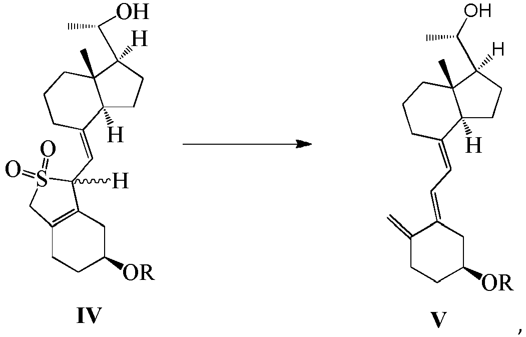 where R is a hydroxyl protection group;
Step 2: reacting compound V with 3-bromomethyl-2,2-dimethyloxirane to give compound VI: 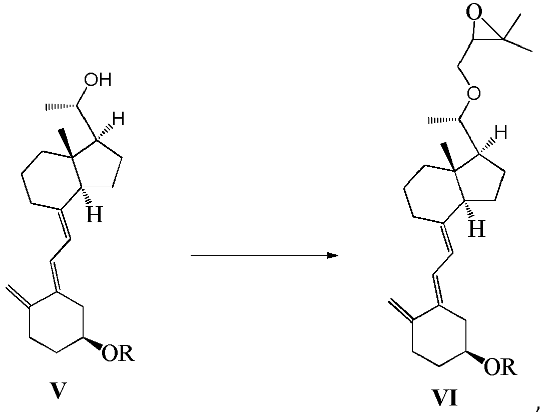 where R is a hydroxyl protection group;
Step 3: converting compound VI into compound VII in the presence of lithium triisobutylhydroborate: 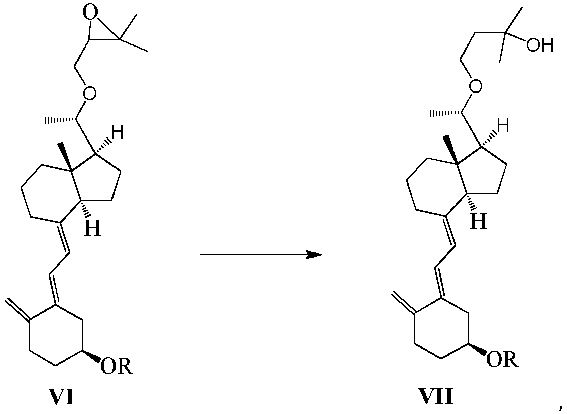 where R is a hydroxyl protection group;
Step 4: reacting compound VII under the action of both *N*-methylmorpholine *N*-oxide and selenium dioxide to give compound VIII: 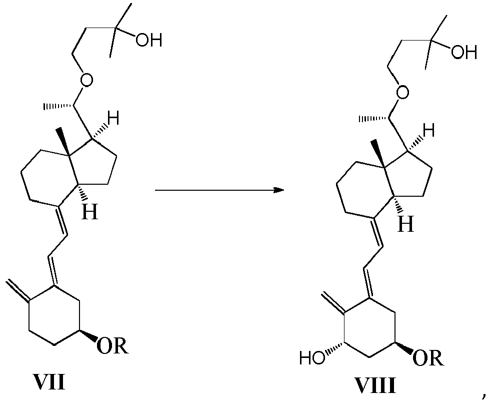 where R is a hydroxyl protection group;
Step 5: de-protecting the hydroxyl protection group of compound VIII to give compound IX: 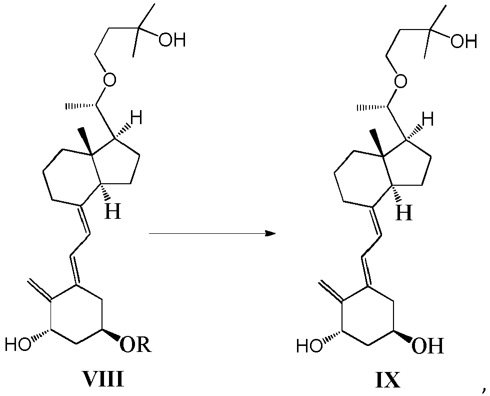 where R is a hydroxyl protection group;
Step 6: conducting a photochemical reaction on compound IX to give Maxacalcitol represented by formula I:

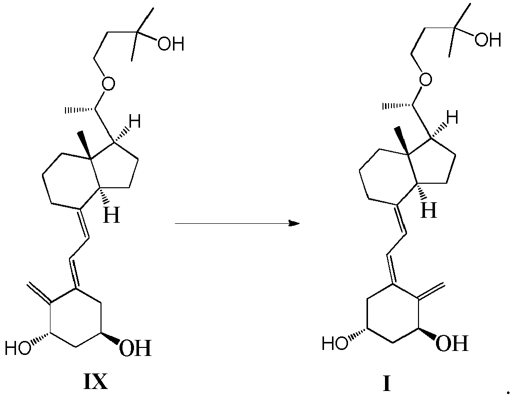

Wherein, the alkali in the step 1 includes sodium bicarbonate or sodium acetate.

In an embodiment of the present invention, a preparation method of Maxacalcitol is provided, which comprises:
conducting a photochemical reaction via uv irradiation on compound IX under the catalysis of 9-acetylanthracene, to overturn the conjugate double bond: 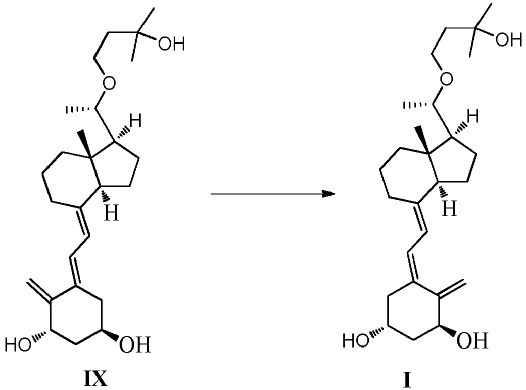 in the reaction, the mass ratio of compound IX to 9-acetylanthracene is preferably 1:(0.05-1), more preferably 1:0.1.

The duration of the reaction can be 0.5 to 5 h, preferably 2 h.

The reaction temperature is preferably 0°C to 10°C.

The reaction can be conducted in a proper organic solvent, the organic solvent can be any proper one, including but not limited to, methanol, ethanol, acetone, dioxane, acetonitrile, THF.

In a further preferred embodiment of the present invention, compound IX can be prepared according to a preparation method as below:
de-protecting compound VIII-1 in the presence of tetrabutylammonium fluoride:

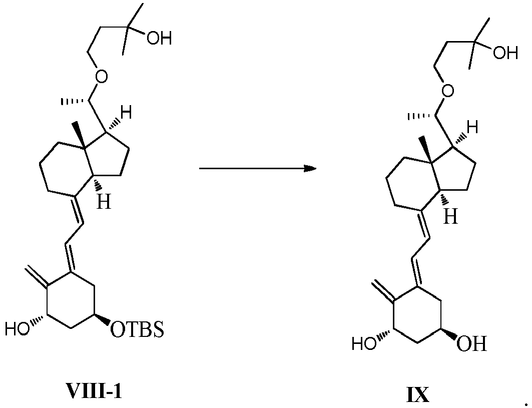

In the reaction, a molar ratio of compound VIII-1 to tetrabutylammonium fluoride is preferably 1:1∼3, more preferably 1:1.5.

The duration of the reaction can be 5 h to 40 h, preferably 10 h.

The reaction temperature is preferably 65°C.

The reaction can be conducted in a proper organic solvent, the organic solvent can be any proper one, including but not limited to, methanol, ethanol, acetone, dioxane, acetonitrile, THF, preferably THF.

In a further preferred embodiment of the present invention, compound VIII-1 can be prepared according to a preparation method as below:
reacting compound VII-1 under the action of both *N*-methylmorpholine *N-*oxide and selenium dioxide:

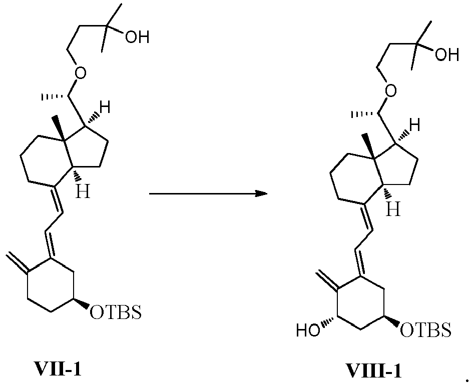

In the reaction, a molar ratio of compound VII-1, *N*-methylmorpholine *N-*oxide and selenium dioxide is preferably 1:(1-3):(0.2-1), more preferably 1:2:0.4.

The duration of the reaction can be 2 h to 24 h, preferably 8 h.

The reaction temperature is preferably 35°C.

In a further preferred embodiment of the present invention, compound VII-1 can be prepared according to a preparation method as below:
reacting compound VI-1 in the presence of lithium triisobutylhydroborate:

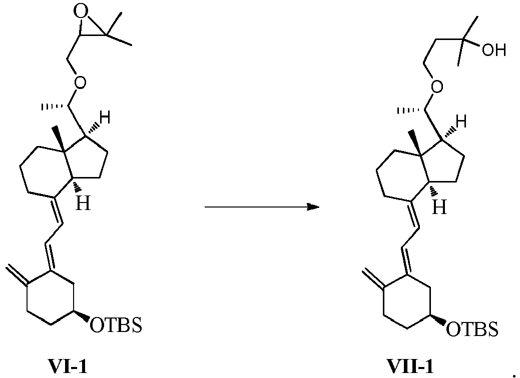

In the reaction, a molar ratio of compound VI-1 to lithium triisobutylhydroborate is preferably 1:(1-3), more preferably 1:1.5.

The duration of the reaction can be 1 h to 10 h, preferably 3 h.

The reaction temperature is preferably 25°C, the solvent is preferably THF.

In a further preferred embodiment of the present invention, compound VI-1 can be prepared according to a preparation method as below:
reacting compound V-1 in the presence of sodium hydride and 3-bromomethyl-2,2-dimethyloxirane:

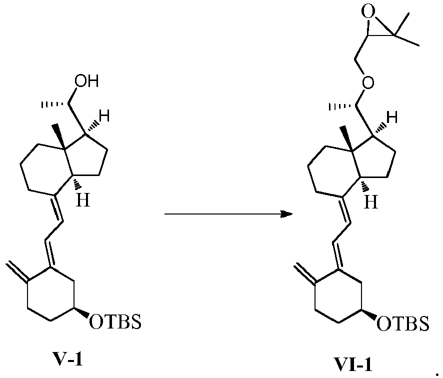

In the reaction, a molar ratio of compound V-1, sodium hydride and 3-bromomethyl-2,2-dimethyloxirane is preferably 1:(1-3):(1-3), more preferably 1:1.2:2.

The duration of the reaction can be 1 h to 10 h, preferably is 5 h.

The reaction temperature is preferably 50°C.

The reaction can be conducted in a proper organic solvent, the organic solvent can be any proper one, including but not limited to, dioxane, acetonitrile, THF, DMF, DMSO, *N,N*-dimethylacetamide or *N*-methylpyrrolidone, etc.

In a further preferred embodiment of the present invention, compound V-1 can be prepared according to a preparation method as below:
converting compound IV-1 into compound V-1 in the presence of sodium bicarbonate:

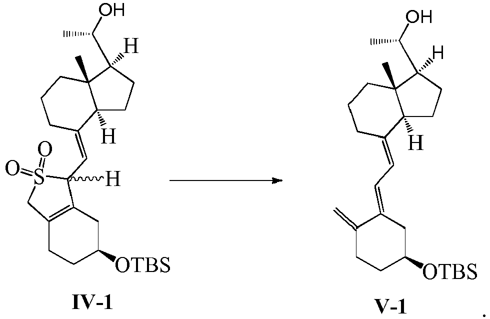

In the reaction, a molar ratio of compound IV-1 to sodium bicarbonate is preferably 1:(1-10), more preferably 1:6.

The duration of the reaction can be 1 h to 24 h, preferably 7 h.

The reaction temperature is preferably 80°C.

The reaction can be conducted in a proper organic solvent, the organic solvent can be any proper one, including but not limited to, 95%(v/v) ethanol, acetonitrile, ethyl acetate or anhydrous ethanol, preferably 95%(v/v) ethanol.

In a further preferred embodiment of the present invention, compound IV-1 can be prepared according to a preparation method as below:
in the presence of a chiral auxiliary reagent (*R*)-2-methyl-CBS-oxazaborolidine, reducing compound III-1 with a borane:

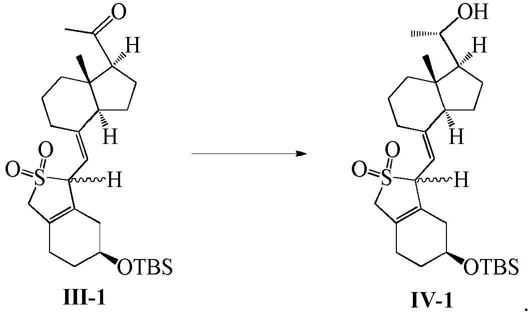

In the reaction, a molar ratio of compound III-1,(*R*)-2-methyl-CBS-oxazaborolidine and borane is preferably 1:(0.1-1):(1-2), more preferably 1:0.6:1.

The reaction temperature can be -60°C to 0°C, preferably -20°C.

The duration of the reaction is preferably 3 h.

In a further preferred embodiment of the present invention, compound III-1 can be prepared according to a preparation method as below:
reacting compound II-1 in the presence of triethylenediamine, 2,2-bipyridine and copper acetate when feeding oxygen:

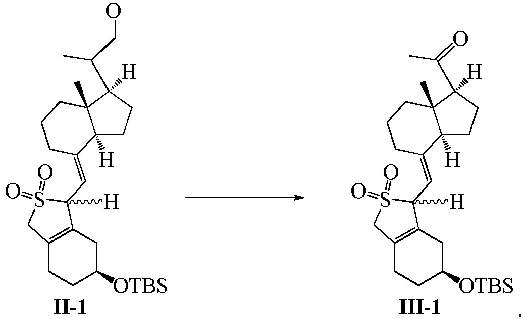

In the reaction, a molar ratio of compound II-1, triethylenediamine, 2,2-bipyridine and copper acetate is preferably 1:(1-2):(0.1-1):(0.1-1), more preferably 1:1:0.2:0.2.

The duration of the reaction can be 1 h to 20 h, preferably 5 h.

The reaction temperature is preferably 45°C.

Wherein, compound II-1 is prepared according to patent US4866048.

The synthetic route of the present invention can be summarized as below:

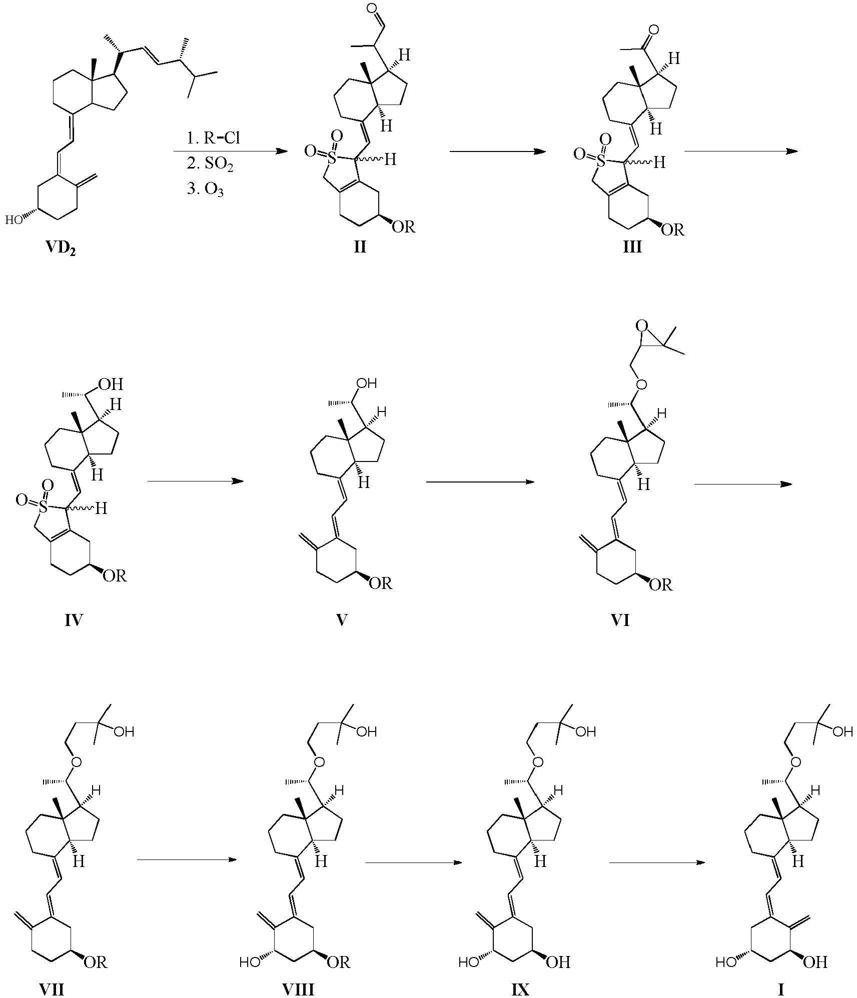

Compared to the prior art, the present invention has the following advantages:
The synthetic process provided by the present invention is crafty-designed, in which vitamin D2 is used as a starting material, compound II is prepared according to the method in US4866048 and then oxidized by oxygen under copper catalysis to deliver compound III. During the oxidation process, due to the protection of sulfur dioxide for the double bond, other side reactions are reduced, which make the yield of oxidation product reach about 80%. However, during the oxidation process of the similar compounds in the prior art, the yield is relative low due to the unstability of the conjugated triple bond, for example, the yield of oxidation reaction mentioned in JP20111573261 is 67% and in reference T.L. 1994, 2295-2298 is 60%-65%. In the present invention, in the presence of a chiral auxiliary reagent, compound III is reduced stereoselectively to give compound IV with single S configuration by employing a borane, and with a high yield of nearly 100%. As sulfur dioxide protects the terminal double bond, side reaction which is the reaction between the borane and the terminal double bond can be efficiently avoided in the reduction reaction, which improves the yield. WO2012/122451 and JP20111573261 conduct the reduction reaction by employing sodium borohydride/lithium aluminum hydride, in which the majority of the product is with R configuration, the yield of product with S configuration is extremely low, furthermore, the products with two configurations have close Rf values, which leads to difficult purification. The present invention protects the double bond with sulfur dioxide, which plays an important role in the oxidation and asymmetric reduction steps, efficiently avoids other side reactions, and improves the reaction yield dramatically. Meanwhile, the following purification becomes much easier since the product with single S configuration is given. The synthesis efficiency is greatly improved, and the process cost is greatly reduced.

### Detailed description of the preferred embodiment

The following examples further illustrate the present invention. It is to be understood that the preparation methods of embodiments are intended to illustrate the present invention in detail, rather than limit the scope of the present invention, any simple modification on the preparation method of the present invention based on the conception of the present invention should belongs to the scope of the present invention.

### Embodiment 1

### Preparation of compound III-1

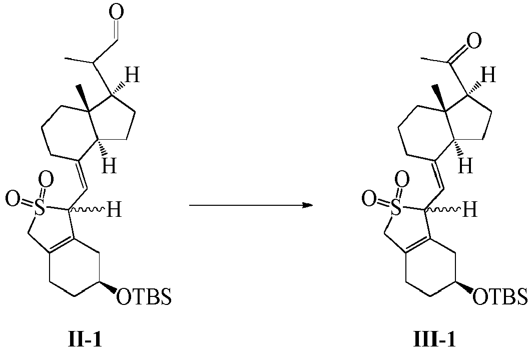

Compound II-1 (50.7 g, 100 mmol) was dissolved in DMF (500 mL), then triethylenediamine (11.2 g, 100 mmol), 2,2-bipyridine (3.12 g, 20 mmol) and copper acetate (3.64 g, 20 mmol) were added separately at room temperature. After adding, the reaction mixture was heated to 45°C at oxygen atmosphere, further stirred for 5 h at this temperature. After the reaction was complete, ethyl acetate was added, the mixture was filtered to remove the insolubles. The filtrate was washed by water for 3 times, dried over anhydrous sodium sulfate, and concentrated under reduced pressure, the oil was isolated and purified to obtain Compound III-1 (39.9 g, yield 81%). The compound was a mixture of two configurations (due to the protection of sulfur dioxide) and can be used directly for the next step. A small amount was taken to be isolated and purified to give a compound with configuration I (having large Rf value) and a compound with configuration II (having small Rf value).

The tested data of ¹H NMR, ¹³C NMR and MS for the two isomers of compound III-1 were as below:

The isomer with small Rf value: ¹H NMR (400 MHz, d-CHCl₃) δ: -0.01 and - 0.00 (each, s, 6H), 0.55 (s, 3H), 0.81(s, 9H), 1.19-2.19(m, 19H), 2.56-2.66(m, 2H) 3.59(s, 2H), 3.95-3.97 (m, 1H), 4.43-4.45 (d, 1H, J=9.6), 4.66-4.68 (d, 1H, J=9.2); ¹³C NMR (100 MHz, d-CHCl₃) δ: -4.7, -4.7, 13.1, 18.1, 22.2, 22.4, 23.7, 24.2, 25.8, 29.6, 30.7, 31.3, 34.3, 39.4, 47.1, 56.3, 58.1, 63.7, 66.5, 67.5, 111.6 , 126.7, 130.5, 149.3, 208.8; MS: m/z (492), Found: 493 (M+H).

The isomer with large Rf value: ¹H NMR (400 MHz, d-CHCl₃) δ:-0.01 and - 0.00 (each, s, 6H), 0.49 (s, 3H), 0.82 (s, 9H), 1.21-2.20 (m, 19H), 2.57-2.60 (m, 1H), 2.67-2.71 (m, 1H), 3.62-3.64 (d, 2H), 3.91-3.93 (m, 1H), 4.55-4.58 (d, 1H, J=9.6), 4.62-4.79 (d, 1H, J=10.0); ¹³C NMR (100 MHz, d-CHCl₃) δ: -4.8, -4.7, 13.4, 18.1, 22.3, 22.5, 23.3, 24.6, 25.8, 29.1, 29.7, 30.9, 31.5, 34.1, 39.1, 46.3, 56.1, 58.2, 63.4, 66.7, 66.8, 111.1, 127.0, 130.2, 148.6, 208.9; MS: m/z (492), Found: 493 (M+H).

### Embodiment 2

### Preparation of compound IV-1

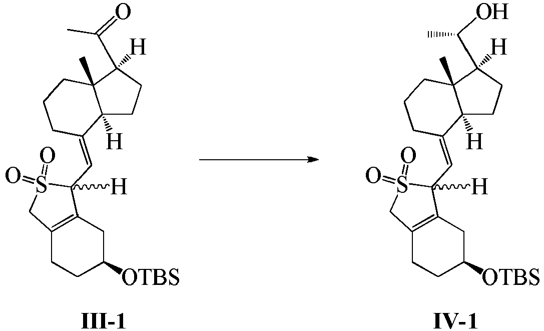

Compound III-1 (49.2 g, 100 mmol) was dissolved in 400 mL anhydrous THF, (*R*)-2-methyl-CBS-oxazaborolidine (1 M, 100 mL) was added slowly at -20°C, followed by dripping BH₃ • THF (1 M, 60 mL) slowly at this temperature, the reaction mixture was further stirred for 1 h after adding, and warmed to room temperature slowly, then 50 mL saturated ammonium chloride solution was added, the mixture was extracted with ethyl acetate, and concentrated under reduced pressure to give 49.5 g oil. The obtained oil was a mixture of two configurations (resulting from the protection of sulfur dioxide, C-20 having single S configuration). A small amount was taken to be isolated and purified to give a compound with configuration I (with large Rf value) and a compound with configuration II (with small Rf value).

The tested data of ¹H NMR, ¹³C NMR and MS for the two isomers of compound IV-1 were as below:

The isomer with small Rf value: ¹H NMR (400 MHz, d-CHCl₃) δ: -0.01 and - 0.00 (each, s, 6H), 0.60 (s, 3H), 0.80 (s, 9H), 1.17-1.20 (m, 6H), 1.48-2.04 (m, 16H), 2.48-2.57 (m, 1H), 3.59 (s, 2H), 3.64-3.68 (m, 1H), 3.94-3.96 (m, 1H), 4.44-4.47 (d, 1H, J=9.2), 4.64-4.66 (d, 1H, J=9.2); ¹³C NMR (100 MHz, d-CHCl₃) δ:-4.7, 12.4, 18.1, 22.0, 23.6, 24.3, 25.0, 25.8, 29.7, 29.7, 30.7, 34.3, 39.3, 45.3, 56.1, 58.1, 58.7, 66.5, 67.6, 70.3, 110.8, 126.5, 130.7, 150.0; MS: m/z =494, Found 495 (M+H).

The isomer with large Rf value: ¹H NMR (400 MHz, d-CHCl₃) δ: -0.01 and - 0.00 (each, s, 6H), 0.52 (s, 3H), 0.82 (s, 9H), 1.18-1.23 (m, 6H), 1.46-2.17 (m, 16H), 2.52-2.55 (m, 1H), 3.60-3.66 (m, 3H), 3.91-3.92 (m, 1H), 4.55-4.58 (d, 1H, J=10.4), 4.73-4.75 (d, 1H, J=10.4); ¹³C NMR (100 MHz, d-CHCl₃) δ: -4.7, 12.4, 18.1, 22.0, 23.6, 24.3, 25.0, 25.8, 29.7, 29.7, 30.7, 34.3, 39.3, 45.3, 56.1, 58.1, 58.7, 66.5, 67.6, 70.3, 110.8, 126.5, 130.7, 150.0; MS: m/z=494, Found 495 (M+H).

### Embodiment 3

### Preparation of compound V-1

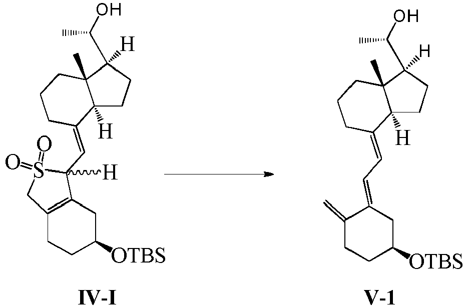

The crude product of compound IV-1 obtained from the previous step was dissolved in 400 mL 95% ethanol, 50 g sodium bicarbonate was added while stirring, then heated to reflux and reacted for further 2∼3 h at this temperature. After the reaction was complete, the ethanol was removed under reduced pressure, ethyl acetate was used to extract. The oil was isolated and purified to give 36.4 g compound V-1, yield 84%.

The tested data of ¹H NMR, ¹³C NMR and MS for compound V-1 were as below:
¹H NMR (400 MHz, CDCl₃) δ: -0.03 (s, 6H, 2SiCH₃), 0.50 (s, 3H, CH₃), 0.82 (s, 9H, 3SiCH₃), 1.16 (d, J=6 Hz, 3H, CH₃), 1.18-1.23 (m, 2H), 1.35-2.22 (m, 13H), 2.38-2.43 (m, 1H), 2.57-2.61 (m, 1H), 2.79-2.83 (m, 1H), 3.64-3.67 (m, 1H, CHOH), 3.78-3.81 (m, 1H, CHOH), 4.58 (s, 1H, =CH₂), 4.86 (s, 1H, =CH₂), 5.81 (d, J=11.6 Hz, 1H, =CH), 6.40 (d, J=11.6 Hz, 1H, =CH); ¹³C NMR (75 MHz, CDCl₃) δ: -4.7, -4.6, 12.7, 18.2, 22.2, 23.2, 23.6, 25.0, 25.9(3C), 28.8, 31.2, 35.2, 37.5, 39.5, 44.9, 56.3, 58.7, 69.4, 70.3, 107.5, 116.5, 119.9, 136.6, 142.9, 150.0; Ms: m/z=430, found 431(M+1).

### Embodiment 4

### Preparation of compound VII-1

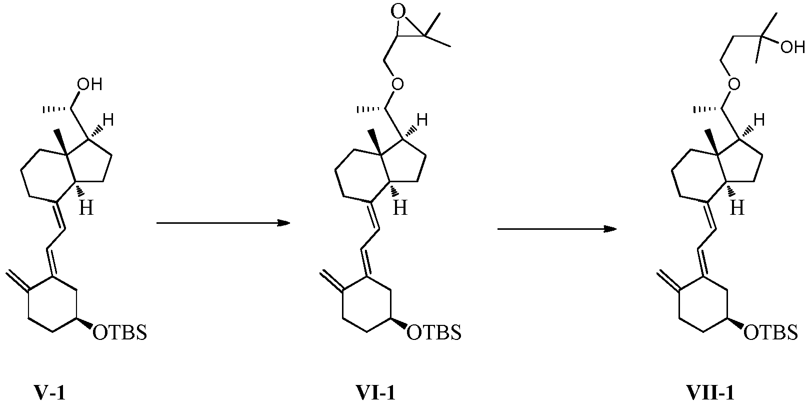

Compound V-1 (43.1 g, 100 mmol) was dissolved in 430 mL anhydrous THF, 60% sodium hydride (4.8 g, 120 mmol) was added at room temperature, then stirred for 0.5 h. 3-bromomethyl-2,2-dimethyloxirane (31 g, 200 mmol) was added and the mixture was heated to reflux and reacted for further 5 h at this temperature. After the reaction was complete, the mixture was cooled to room temperature, lithium triisobutylhydroborate (150 mL, 1 M in THF) was added, and then further stirred for 3 h after adding. Saturated ammonium chloride solution 100 mL was added, the mixture was extracted with ethyl acetate, and concentrated, the obtained oil was isolated and purified to give 40.3 g compound VII-1, yield 78%.

The tested data of ¹H NMR, ¹³C NMR and MS for compound VII-1 were as below:
¹H NMR (400 MHz, CDCl₃) δ:-0.07 (s, 3H, SiCH₃), -0.06 (s, 3H, SiCH₃), 0.48 (s, 3H, CH₃), 0.83 (s, 9H, 3SiCH₃), 0.72-0.97 (m, 2H), 1.13 (d, J=6 Hz, 3H, CH₃), 1.17 (s, 3H, CH₃), 1.18 (s, 3H, CH₃), 1.19-1.27 (m, 2H), 1.35-2.22 (m, 13H,), 2.39-2.42 (m, 1H), 2.56-2.61 (m, 1H), 2.78-2.82 (m, 1H), 3.17-3.21 (m, 1H, CHOH), 3.41-3.44 (m, 1H, CHOH), 3.77-3.81 (m, 3H, OH and CHOH), 4.58 (s, 1H, =CH₂), 4.86 (s, 1H, =CH₂), 5.80 (d, J=11.6 Hz, 1H, =CH), 6.39 (d, J=11.6 Hz, 1H, =CH); ¹³C NMR (75 MHz, CDCl₃) δ: -4.7, -4.6, 12.7, 18.2, 18.8, 22.2, 23.2, 25.9(3C), 26.0, 28.8, 29.1, 29.4, 31.2, 35.2, 37.5, 39.6, 41.5, 44.7, 56.2, 57.1, 65.6, 69.4, 70.5, 79.0, 107.6, 116.5, 119.9, 136.5, 142.8, 150.0; Ms: m/z=516, found 517 (M+1).

### Embodiment 5

### Preparation of compound VIII-1

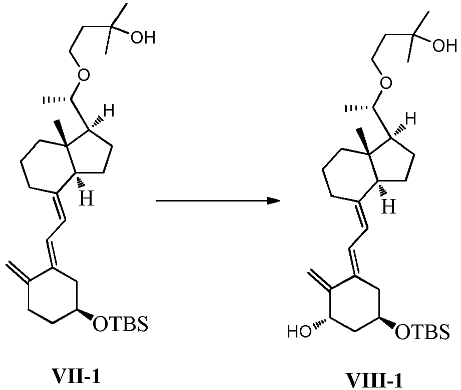

Compound VII-1 (41.2 g, 80 mmol) was dissolved in 500 mL dichloromethane, then *N*-methylmorpholine *N*-oxide (18.7 g, 160 mmol) and selenium dioxide (3.55 g, 32 mmol) were added, argon was introduced to replace the air in the reaction flask. The reaction mixture was heated to reflux, then further reacted for 5-6 h at this temperature. After the reaction was complete, the mixture was cooled to room temperature, water was added, and dichloromethane was used to extract. The organic phase was concentrated under reduced pressure, then the residue was isolated and purified by column chromatography, elution system was petroleum ether:ethyl acetate = 10:1, to obtain Compound VIII-1 (15.7 g), yield 37%.

The tested data of ¹H NMR, ¹³C NMR and MS for compound VIII-1 were as below:
¹H NMR (400 MHz, CDCl₃) δ:-0.01 (s, 6H, 2SiCH₃), 0.46 (s, 3H, CH₃), 0.83 (s, 9H, 3SiCH₃), 1.12 (d, J=6 Hz, 3H, CH₃), 1.16 (s, 3H, CH₃), 1.17 (s, 3H, CH₃), 1.18-1.27 (m, 2H), 1.42-1.97 (m, 15H), 2.34-2.47 (m, 1H), 2.77-2.81 (m, 1H), 3.16-3.20 (m, 1H, CHOH), 3.41-3.44 (m, 1H, CHOH), 3.75-3.80 (m, 2H, OH and CHOH), 4.11-4.14 (m, 1H, CHOH), 4.41-4.44 (m, 1H, CHOH), 4.88 (s, 1H, =CH₂), 4.99 (s, 1H, =CH₂), 5.78 (d, J=11.6 Hz, 1H, =CH), 6.42 (d, J=11.6 Hz, 1H, =CH); ¹³C NMR (75 MHz, CDCl₃) δ: -4.8, -4.7, 12.6, 18.1, 18.8, 22.2, 23.2, 25.9(3C), 26.0, 28.9, 29.1, 29.4, 37.0, 39.6, 41.5, 42.9, 44.8, 56.2, 57.1, 65.6, 66.8, 70.5, 70.6, 79.0, 107.7, 116.6, 122.2, 134.6, 143.3, 153.1; Ms: m/z=532, found 555 (M+Na).

### Embodiment 6

### Preparation of compound IX-1

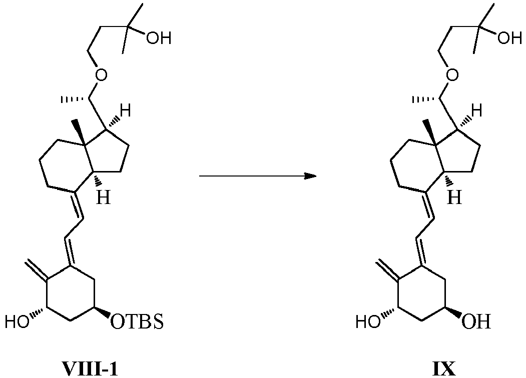

Compound VIII-1 (26.6 g, 50 mmol) was dissolved in 270 mL THF, Bu₄NF (19.5 g, 75 mmol) was added, then the reaction mixture was heated to reflux and stirred further for 7-8 h at this temperature. After the reaction was complete, the heating was stopped and the mixture was cooled to room temperature, THF was removed under reduced pressure, ethyl acetate was used to extract. After concentration under reduced pressure, the obtained oil was isolated and purified to give 18 g compound IX, yield 86%.

The tested data of ¹H NMR, ¹³C NMR and MS for compound IX were as below:
¹H NMR (400 MHz, CDCl₃) δ: 0.54 (s, 3H, CH₃), 1.19 (s, J=5.6 Hz, 3H, CH₃), 1.23 (s, 6H, 2CH₃), 1.24 -1.37 (m, 2H), 1.48-2.08 (m, 13H), 2.24-2.30 (m, 1H), 2.44(s, br, 1H, OH), 2.65 (s, br, 1H, OH), 2.81-2.88 (m, 2H), 3.24-3.27 (m, 1H), 3.46-3.51 (m, 1H, CHOH), 3.82-3.90 (m, 2H, OH and CHOH), 4.19-4.23 (m, 1H, CHOH), 4.47-4.49 (m, 1H, CHOH), 4.96 (s, 1H, =CH₂), 5.10 (s, 1H, =CH₂), 5.89 (d, J=11.2 Hz, 1H, =CH), 6.55 (d, J=11.2 Hz, 1H, =CH); ¹³C NMR (75 MHz, CDCl₃) δ:12.8, 18.9, 22.2, 23.2, 25.8, 28.9, 29.1, 29.2, 38.7, 39.5, 41.5, 41.9, 44.8, 56.2, 57.1, 65.5, 65.6, 70.7, 70.8, 78.9, 109.5, 116.5, 122.8, 133.5, 144.0, 151.8; Ms: m/z=418, found 441 (M+Na).

### Embodiment 7

### Preparation of compound I

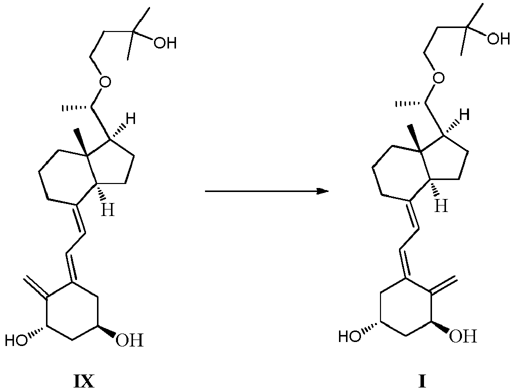

Compound IX (21 g) was dissolved in 3000 mL acetone, 9-acetylanthracene (2.1 g) was added. Turn on the cooling equipment, cool to below 5°C. Turn on the photochemical reaction instrument, conduct the uv irradiation reaction at 350nm. After 0.5 h, sample was taken to monitor the reaction, and duration of the reaction was estimated according to the monitor result, which is about 2 h. After the reaction was complete, acetone was concentrated, the obtained residue was eluted through column chromatography, elution system is petroleum ether:ethyl acetate = 1:1, to obtain 19.3 g Compound I, yield 92%.

The tested data of ¹H NMR, ¹³C NMR and MS for compound I were as below:
¹H NMR (400 MHz, d-DMSO) δ: 0.49 (s, 3H, CH₃), 1.08 (s, 6H, 2CH₃), 1.09 (d, J=1.6 Hz, 3H, CH₃), 1.22-1.28 (m, 1H), 1.39-1.65 (m, 10H), 1.79-1.84 (m, 3H), 1.93-1.99 (m, 1H), 2.15-2.20 (m, 1H), 2.35-2.37 (m, 1H), 2.78-2.81 (m, 1H), 3.18-3.21 (m, 1H), 3.25-3.31 (m, 1H), 3.60 (q, J=7.6Hz, 1H), 3.99-4.04 (m, 1H, CHOH), 4.12(s, 1H, OH), 4.18-4.21 (m, 1H, CHOH), 4.54 (d, J=4 Hz, 1H, OH), 4.76 (s, 1H, =CH₂), 4.86 (d, J=4.4 Hz, 1H, OH), 5.23 (s, 1H, =CH₂), 5.99 (d, J=11.2 Hz, 1H, =CH), 6.18 (d, J=11.2 Hz, 1H, =CH); ¹³C NMR(75 MHz, d-DMSO) δ: 12.3, 19.1, 21.8, 22.9, 24.7, 28.3, 29.6, 29.7, 38.9, 43.1, 43.2, 44.1, 44.9, 55.5, 56.8, 64.3, 65.1, 68.2, 68.4, 76.7, 109.8, 117.8, 122.4, 135.9, 139.6, 149.5; Ms: m/z=418, found 441 (M+Na).

## Claims

1. A compound represented by formula III: 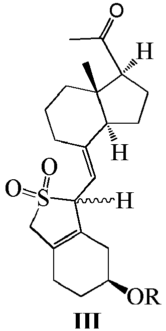 where R is H or a hydroxyl protection group; the hydroxyl protection group is selected from a *t*-butyldimethylsilyl, a trimethylsilyl, a triethylsilyl, a *t-*butyldiphenylsilyl or a triisoprolylsilyl.

2. A preparation method for the compound III according to claim 1, in the presence of a catalyst, oxidating compound II with an oxidizing agent to give compound III:

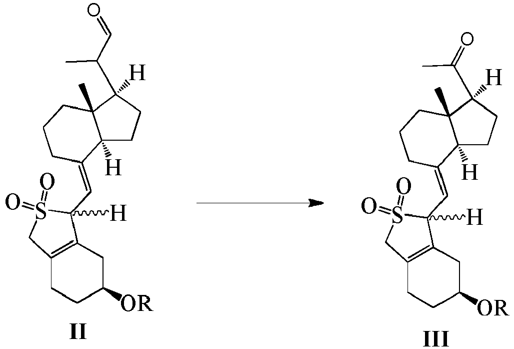

3. The preparation method according to claim 3, wherein the oxidizing agent is selected from oxygen; the catalyst is selected from a copper catalyst.

4. The preparation method according to claim 3, wherein the catalyst is 2,2-bipyridine copper complex.

5. A compound represented by formula IV: 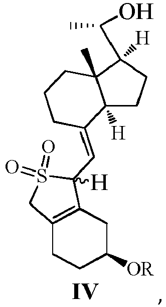 where R is defined in claim 1.

6. A preparation method for the compound IV according to claim 5, comprising in the presence of a chiral auxiliary reagent, reducing compound III with a borane to give compound IV: 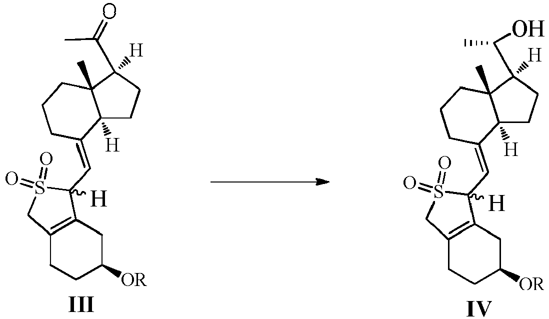 where R is defined in claim 1.

7. The preparation method according to claim 6, wherein the chiral auxiliary reagent is selected from (*R*)-2-methyl-CBS-oxazaborolidine, (*R*)-2-ethyl-CBS-oxazaborolidine or (*R*)-2-isopropyl-CBS-oxazaborolidine.

8. The preparation method according to claim 6, wherein the borane is selected from BH₃, borane-tetrahydrofuran complex, borane-triethylamine complex, borane-ethyl ether complex, borane-methyl sulfide complex or borane-*N,N*-diethylaniline complex.

9. The preparation method according to at least one of claim 6-8, wherein a molar ratio of the compound III, the chiral auxiliary reagent and the borane is 1 :(0.1∼1):(1∼2), optionally, the molar ratio of the compound III, the chiral auxiliary reagent and the borane is 1:0.6:1; the reaction temperature is -60°C to 0°C, optionally, the reaction temperature is -20°C.

10. A compound represented by formula VI: 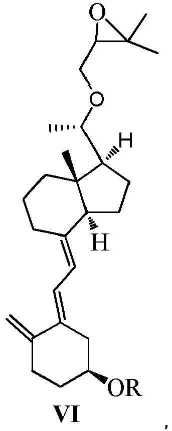 where R is defined in claim 1.

11. A preparation method for the compound VI according to claim 10, comprising:
Step 1: converting compound IV into compound V under alkaline condition: 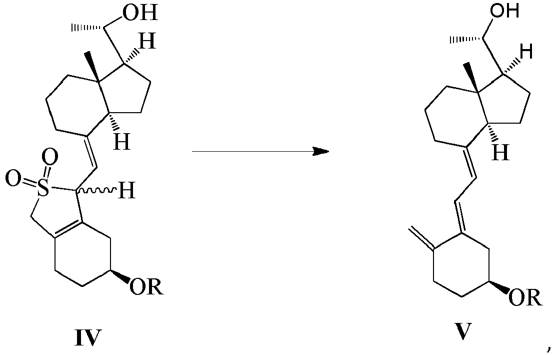
Step 2: reacting compound V with 3-bromomethyl-2,2-dimethyloxirane to give compound VI: 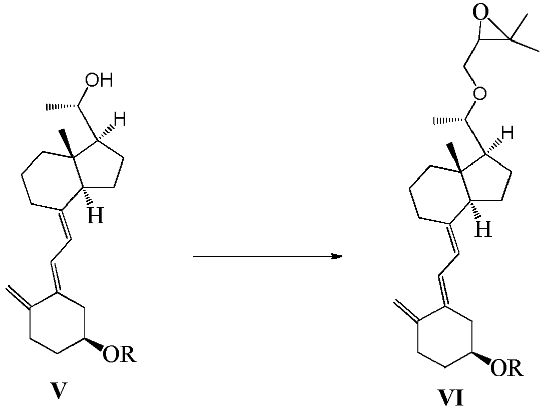 where R is defined in claim 1 except for H.

12. The preparation method for the compound VI according to claim 11, further comprising de-protecting the hydroxyl protection group R of the compound VI obtained in the step 2 to give compound VI: 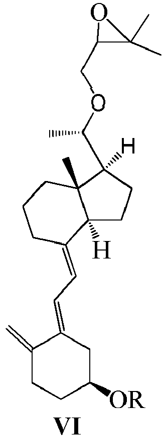 where R is H.

13. A preparation method for Maxacalcitol represented by formula I: 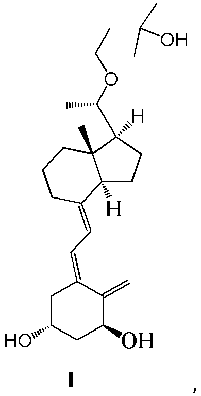 which comprises:
Step 1: converting compound IV into compound V under alkaline condition: 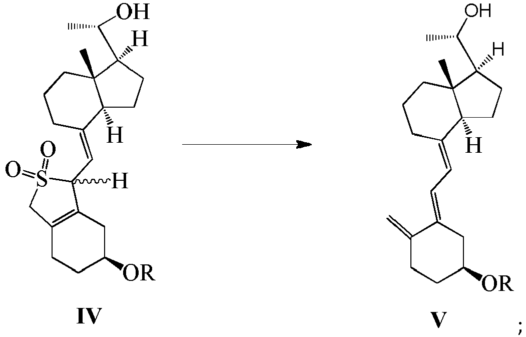
Step 2: reacting compound V with 3-bromomethyl-2,2-dimethyloxirane to give compound VI: 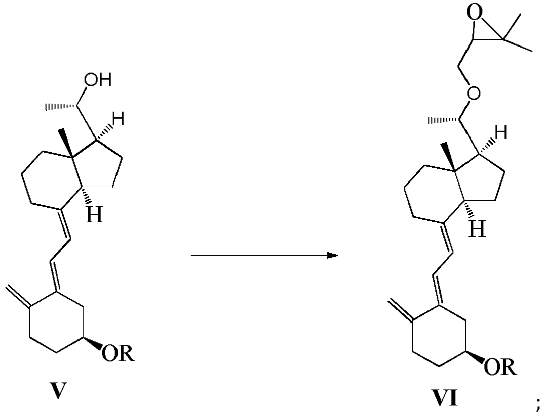
Step 3: converting compound VI into compound VII in the presence of lithium triisobutylhydroborate: 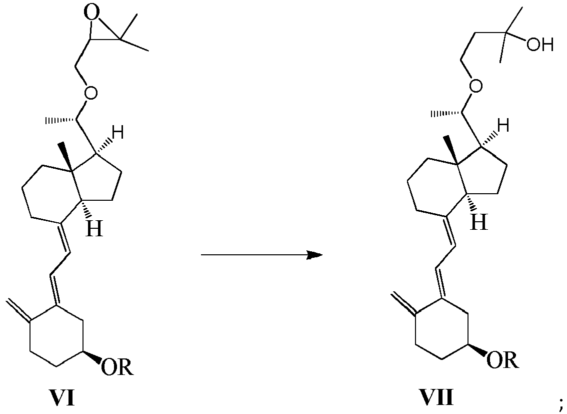
Step 4: reacting compound VII under the action of both *N*-methylmorpholine *N-*oxide and selenium dioxide to give compound VIII: 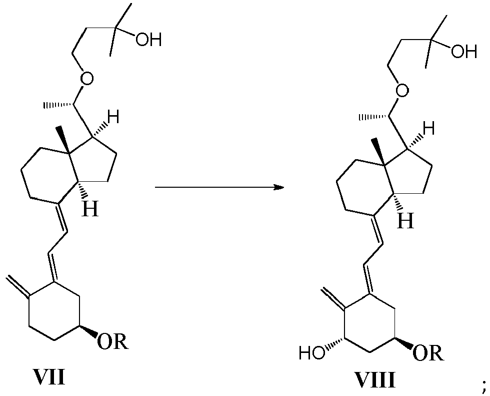
Step 5: de-protecting the hydroxyl protection group of compound VIII to give compound IX: 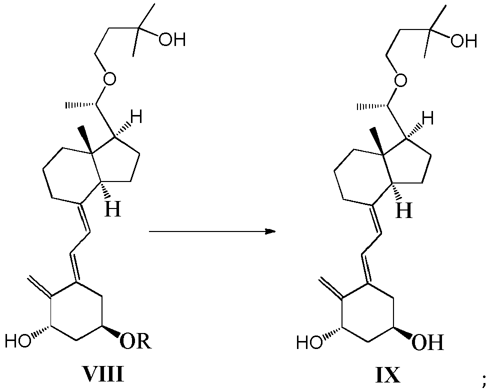
Step 6: conducting a photochemical reaction on compound IX to give Maxacalcitol represented by formula I: 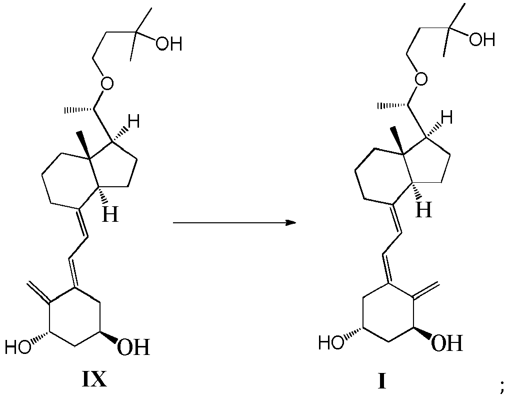
where R is defined in claim 1 except for H.

14. The preparation method according to claim 13, which further comprises: in the presence of a chiral auxiliary reagent, reducing compound III with a borane to give compound IV: 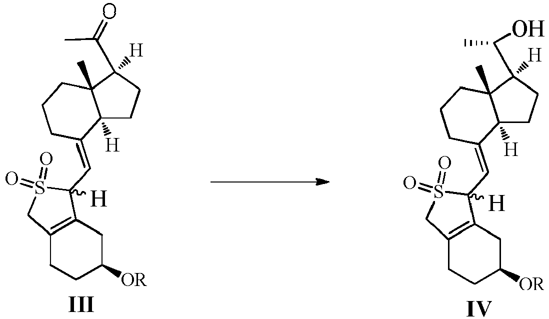 wherein the reaction parameters are defined as at least one of claim 6-9.

15. The preparation method according to claim 14, which further comprises: in the presence of a catalyst, oxidating compound II with an oxidizing agent to give compound III: 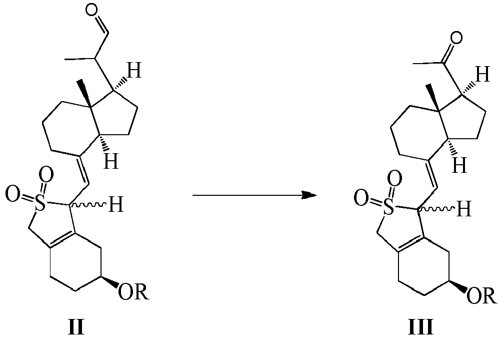 wherein the reaction parameters are defined as at least one of claim 3-4.

16. A use of the compound III according to claim 1 in preparing Maxacalcitol.

## Patentansprüche

1. Verbindung, dargestellt durch die Formel III: 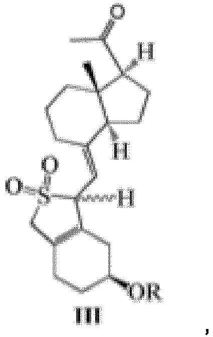 wobei R H oder eine Hydroxylschutzgruppe ist; wobei die Hydroxylschutzgruppe ausgewählt ist aus *t*-Butyldimethylsilyl, einem Trimethylsilyl, einem Triethylsilyl, einem t-Butyldiphenylsilyl oder einem Triisoprolylsilyl.

2. Verfahren zur Zubereitung der Verbindung III nach Anspruch 1 in Gegenwart eines Katalysators, wobei die Verbindung II mit einem Oxidationsmittel oxidiert wird, so dass die Verbindung III resultiert:

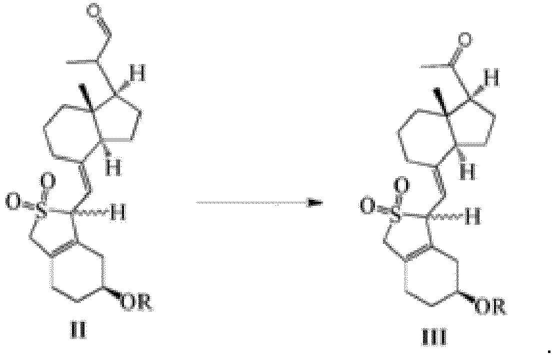

3. Verfahren zur Zubereitung nach Anspruch 3, wobei das Oxidationsmittel ausgewählt ist aus Sauerstoff; wobei der Katalysator aus einem Kupferkatalysator ausgewählt ist.

4. Verfahren zur Zubereitung nach Anspruch 3, wobei der Katalysator ein 2,2-Bipyridin-Kupferkomplex ist.

5. Verbindung, dargestellt durch die Formel IV: 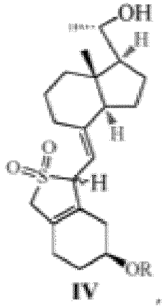 wobei R in Anspruch 1 definiert ist.

6. Verfahren zur Zubereitung der Verbindung IV nach Anspruch 5, wobei dieses in Gegenwart eines Reagens eines chiralen Hilfsstoffs das Reduzieren der Verbindung III mit einem Boran umfasst, so dass die Verbindung IV resultiert: 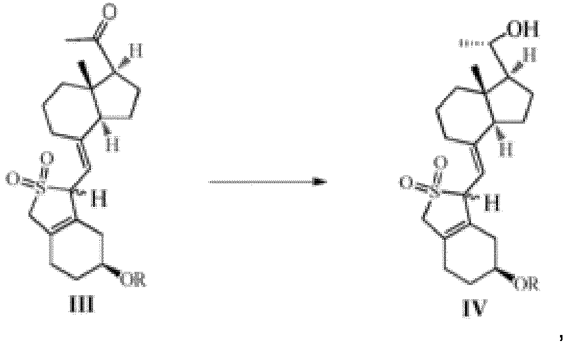 wobei R in Anspruch 1 definiert ist.

7. Verfahren zur Zubereitung nach Anspruch 6, wobei das Reagens eines chiralen Hilfsstoffs ausgewählt ist aus (*R*)-2-Methyl-CBS-oxazaborolidin, (*R*)-2-Ethyl-CBS-oxazaborolidin oder (*R*)-2-Isopropyl-CBS-oxazaborolidin.

8. Verfahren zur Zubereitung nach Anspruch 6, wobei das Boran ausgewählt ist aus BH₃, einem Boran-Tetrahydrofuran-Komplex, einem Boran-Triethylamin-Komplex, einem Boran-Ethylether-Komplex, einem Boran-Methylsulfid-Komplex oder einem Boran-*N,N*-Diethylanilin-Komplex.

9. Verfahren zur Zubereitung nach wenigstens einem der Ansprüche 6 bis 8, wobei ein Molverhältnis der Verbindung III, des Reagens eines chiralen Hilfsstoffs und des Borans 1:(0,1∼1):(1∼2) beträgt, wobei das Molverhältnis der Verbindung III, des Reagens eines chiralen Hilfsstoffs und des Borans optional 1:0,6:1 beträgt; wobei die Reaktionstemperatur -60 °C bis 0 °C beträgt, wobei die Reaktionstemperatur optional -20 °C beträgt.

10. Verbindung, dargestellt durch die Formel VI: 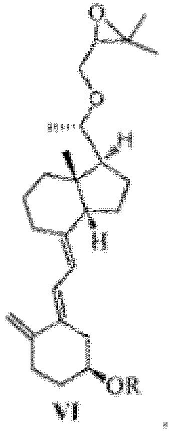 wobei R in Anspruch 1 definiert ist.

11. Verfahren zur Zubereitung der Verbindung der Verbindung VI nach Anspruch 10, umfassend:
Schritt 1: Umwandeln der Verbindung IV in Verbindung V unter alkalischen Bedingungen: 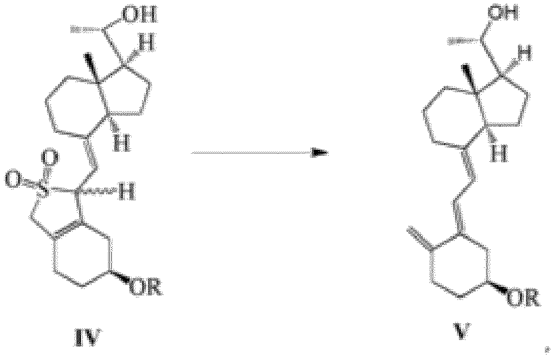
Schritt 2: Reagieren von Verbindung V mit 3-Brommethyl-2,2-dimethyloxiran, so dass Verbindung VI resultiert: 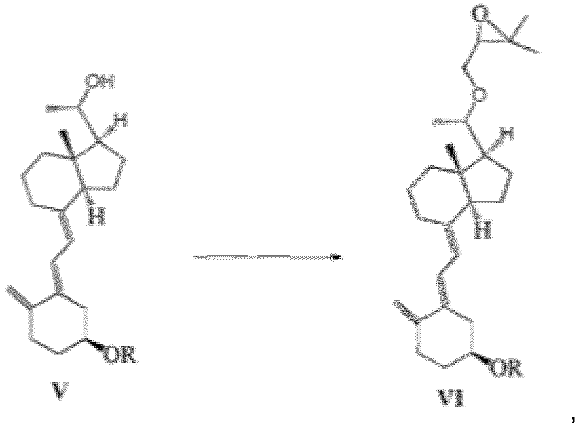 wobei R in Anspruch 1 definiert ist, mit Ausnahme von H.

12. Verfahren zur Zubereitung der Verbindung der Verbindung VI nach Anspruch 11, ferner umfassend das Entschützen der Hydroxylschutzgruppe R der in Schritt 2 erhaltenen Verbindung VI, um Verbindung VI zu erhalten: 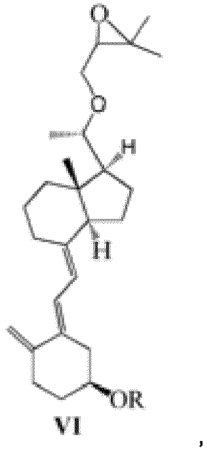 wobei R H ist.

13. Verfahren zur Zubereitung von Maxacalcitol, dargestellt durch Formel I: 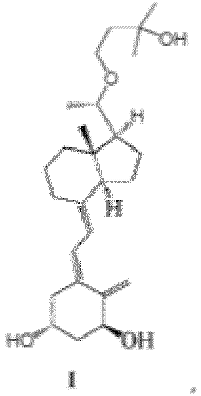 umfassend:
Schritt 1: Umwandeln der Verbindung IV in Verbindung V unter alkalischen Bedingungen: 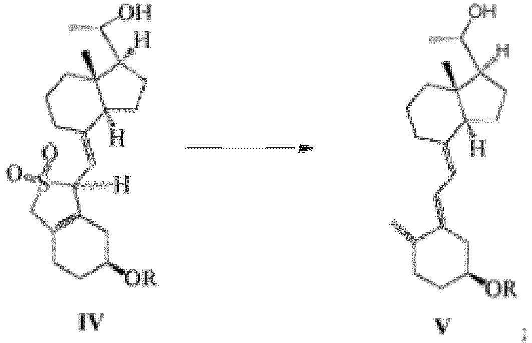
Schritt 2: Reagieren von Verbindung V mit 3-Brommethyl-2,2-dimethyloxiran, so dass Verbindung VI resultiert: 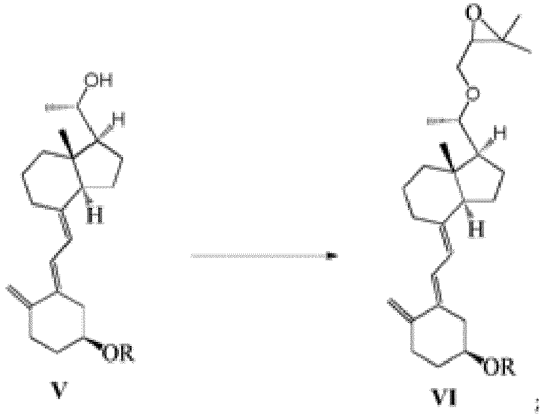
Schritt 3: Umwandeln von Verbindung VI in Verbindung VII in Gegenwart von Lithiumtriisobutylhydroborat: 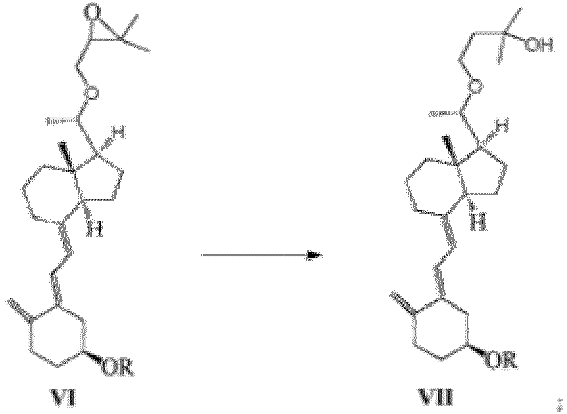
Schritt 4: Reagieren von Verbindung VII unter der Wirkung von sowohl N-Methylmorpholin *N*-Oxid und Selenoxid, um Verbindung VIII zu erhalten: 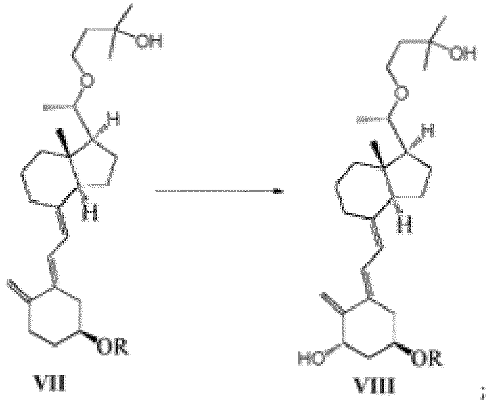
Schritt 5: Entschützen der Hydroxylschutzgruppe von Verbindung VIII, um Verbindung IX zu erhalten: 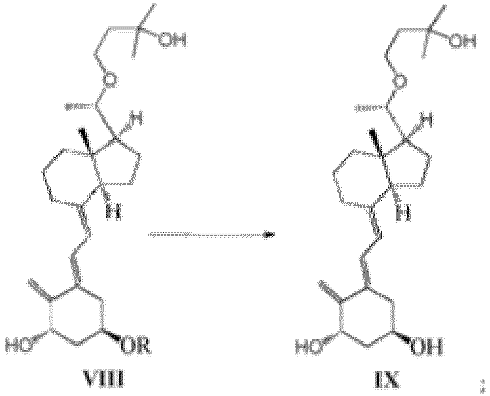
Schritt 6: Durchführen einer photochemischen Reaktion an Verbindung IX, um das durch Formel I dargestellte Maxacalcitol zu erhalten: 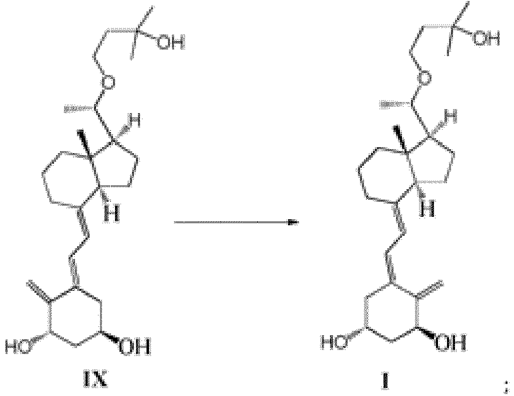 wobei R in Anspruch 1 definiert ist, mit Ausnahme von H.

14. Verfahren zur Zubereitung nach Anspruch 13, das ferner folgendes umfasst: in Gegenwart eines Reagens eines chiralen Hilfsstoffs das Reduzieren der Verbindung III mit einem Boran umfasst, so dass die Verbindung IV resultiert: 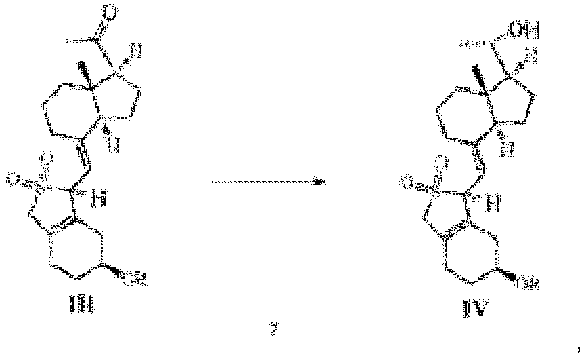 wobei die Reaktionsparameter gemäß wenigstens einem der Ansprüche 6 bis 9 definiert sind.

15. Verfahren zur Zubereitung nach Anspruch 14, das ferner folgendes umfasst: in Gegenwart eines Katalysators das Oxidieren von Verbindung II mit einem Oxidationsmittel, um die Verbindung III zu erhalten: 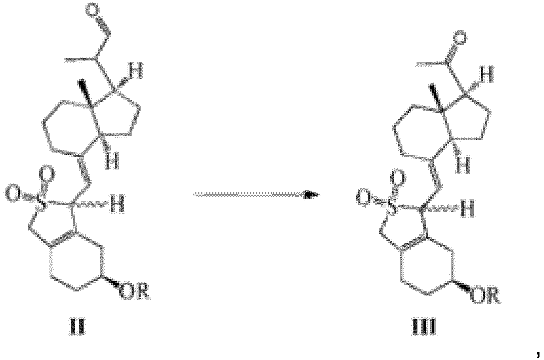 wobei die Reaktionsparameter gemäß wenigstens einem der Ansprüche 3 bis 4 definiert sind.

16. Verwendung der Verbindung III nach Anspruch 1 bei der Zubereitung von Maxacalcitol.

## Revendications

1. Composé représenté par la formule III : 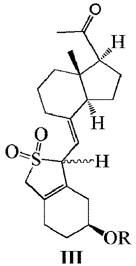 où R est H ou un groupe de protection hydroxyle ; le groupe de protection hydroxyle étant choisi parmi un *t*-butyldiméthylsilyle, un triméthylsilyle, un triéthylsilyle, un *t*-butyldiphénylsilyle ou un triisoprolylsilyle.

2. Procédé de préparation du composé III selon la revendication 1, en présence d'un catalyseur, oxydant le composé II avec un agent oxydant pour donner le composé III :

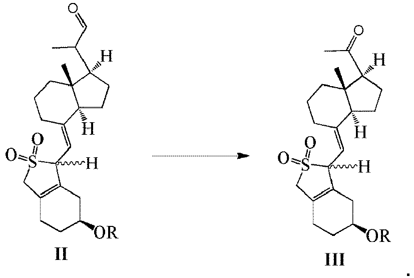

3. Procédé de préparation selon la revendication 3, l'agent oxydant étant choisi parmi l'oxygène ; le catalyseur étant choisi parmi un catalyseur au cuivre.

4. Procédé selon la revendication 3, le catalyseur étant un complexe de cuivre 2,2-bipyridine.

5. Composé représenté par la formule IV : 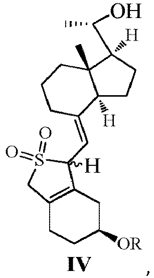 où R est défini dans la revendication 1.

6. Procédé de préparation du composé IV selon la revendication 5, comprenant, en présence d'un réactif auxiliaire chiral, l'étape consistant à réduire le composé III avec un borane pour donner le composé IV : 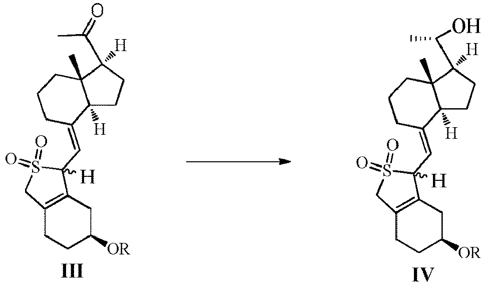 où R est défini dans la revendication 1.

7. Procédé de préparation selon la revendication 6, le réactif auxiliaire chiral étant choisi parmi la (*R*)-2-méthyl-CBS-oxazaborolidine, la (*R*)-2-éthyl-CBS-oxazaborolidine ou la (R)-2-isopropyl-CBS-oxazaborolidine.

8. Procédé de préparation selon la revendication 6, le borane étant choisi parmi BH₃, le complexe borane-tétrahydrofurane, le complexe borane-triéthylamine, le complexe borane-éther éthylique, le complexe borane-sulfure de méthyle ou le complexe borane-*N,N*-diéthylaniline.

9. Procédé de préparation selon au moins l'une des revendications 6 à 8, un rapport molaire du composé III, du réactif auxiliaire chiral et du borane étant de 1:(0,1∼1):(1∼2), facultativement, le rapport molaire du composé III, du réactif auxiliaire chiral et du borane étant de 1:0,6:1 ; la température de réaction est de -60 °C à 0 °C, éventuellement, la température de réaction étant de -20 °C.

10. Composé représenté par la formule VI : 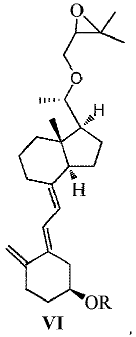 où R est défini dans la revendication 1.

11. Procédé de préparation du composé VI selon la revendication 10, comprenant :
Étape 1 : convertir le composé IV en composé V dans des conditions alcalines : 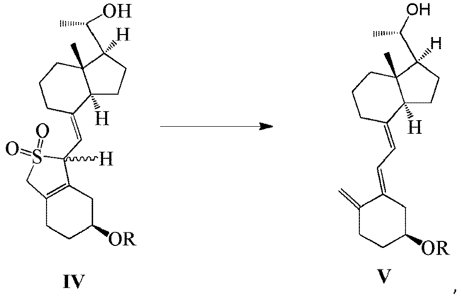
Étape 2 : faire réagir le composé V avec le 3-bromométhyl-2,2-diméthyloxirane pour donner le composé VI : 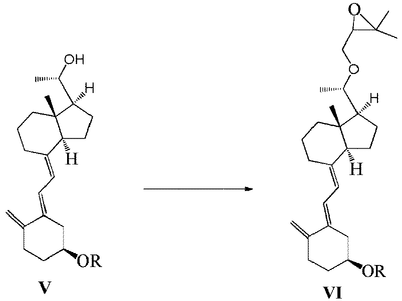 où R est défini dans la revendication 1, à l'exception de H.

12. Procédé de préparation pour le composé VI selon la revendication 11, comprenant en outre la déprotection du groupe de protection hydroxyle R du composé VI obtenu à l'étape 2 pour donner le composé VI : 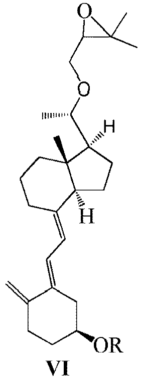 où R est H.

13. Procédé de préparation de Maxacalcitol représenté par la formule I : 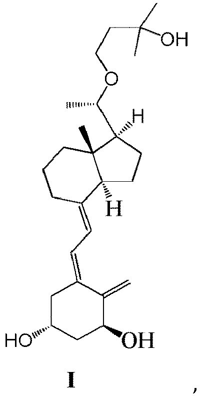 qui comprend :
Étape 1 : convertir le composé IV en composé V dans des conditions alcalines : 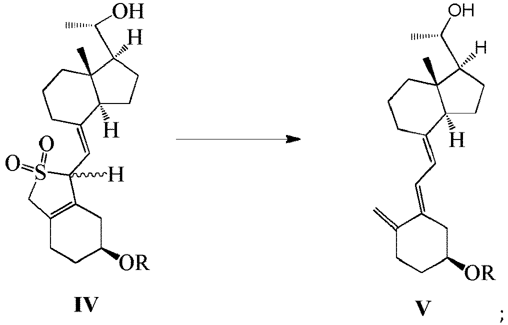
Étape 2 : faire réagir le composé V avec le 3-bromométhyl-2,2-diméthyloxirane pour donner le composé VI : 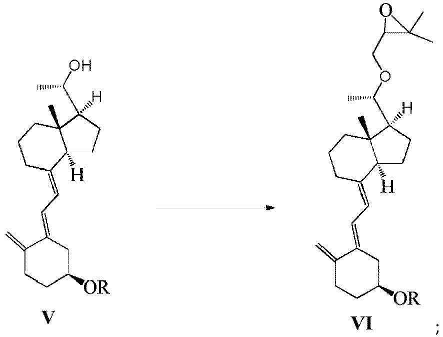
Étape 3 : convertir le composé VI en composé VII en présence de triisobutylhydroborate de lithium : 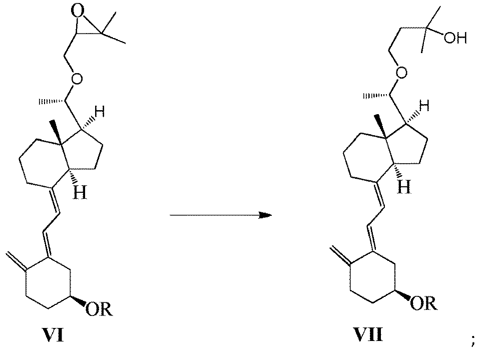
Étape 4 : faire réagir le composé VII sous l'action de *N*-méthylmorpholine *N-*oxyde et de dioxyde de sélénium pour donner le composé VIII : 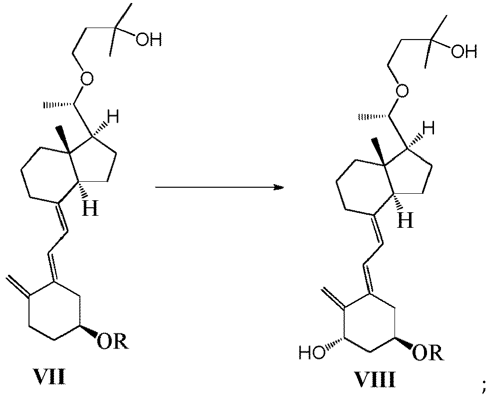
Étape 5 : déprotéger le groupe de protection hydroxyle du composé VIII pour donner le composé IX : 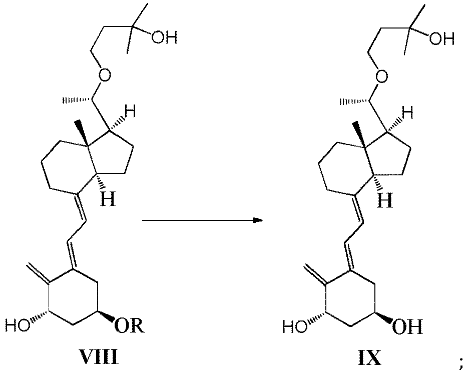
Étape 6 : effectuer une réaction photochimique sur le composé IX pour donner le Maxacalcitol représenté par la formule I : 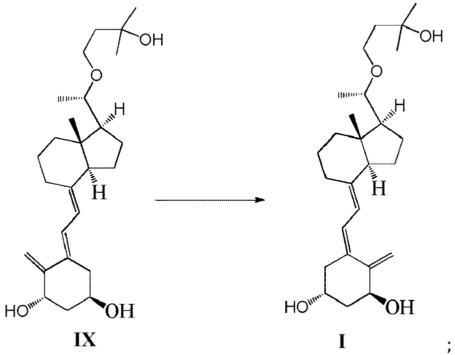 où R est défini dans la revendication 1, à l'exception de H.

14. Procédé de préparation selon la revendication 13, qui comprend en outre l'étape consistant à : en présence d'un réactif auxiliaire chiral, réduire le composé III avec un borane pour donner le composé IV : 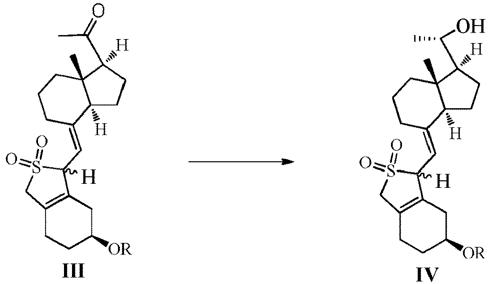 les paramètres de réaction étant définis comme étant au moins l'un de la revendication 6 à 9.

15. Procédé de préparation selon la revendication 14, qui comprend en outre l'étape consistant à : en présence d'un catalyseur, oxyder le composé II avec un agent oxydant pour donner le composé III : 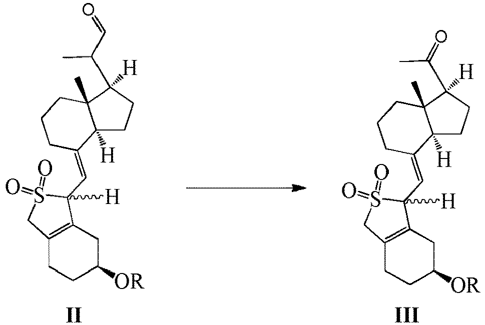 les paramètres de réaction étant définis comme étant au moins l'un de la revendication 3 à 4.

16. Utilisation du composé III selon la revendication 1 dans la préparation de Maxacalcitol.
